# FASCICULE DE BREVET EUROPEEN

(11) **EP 2 911 680 B1**
(45) Date de publication et mention de la délivrance du brevet: **28.02.2018**
(21) Numéro de dépôt: 13815019.8
(22) Date de dépôt: 25.10.2013
(51) Int. Cl.: A61K 36/55, A61P 17/00, A61P 31/00

(54) **UTILISATION D'UN EXTRAIT DE LIN PROVENANT DE L'HYDROLYSE DES PROTÉINES DE LIN, EN TANT QU'AGENT ACTIF ANTIMICROBIEN**
VERWENDUNG EINES FLACHSEXTRAKTES AUS DER HYDROLYSE VON FLACHSPROTEINEN ALS AKTIVES ANTIMIKROBIELLES MITTEL
USE OF A FLAX EXTRACT, ORIGINATING FROM THE HYDROLYSIS OF FLAX PROTEINS, AS AN ACTIVE ANTIMICROBIAL AGENT

(30) Priorité: 26.10.2012 FR 1260235
(43) Date de publication de la demande: 02.09.2015
(73) Titulaire: ISP INVESTMENT INC., Wilmington, DE 19805 (US)
(72) Inventeur: BOTTO, Jean-Marie, F-06560 Garbejaïre (FR); DOMLOGE, Nouha, F-06560 Valbonne (FR); PORTOLAN, Frédérique, F-06560 Valbonne (FR)
(74) Mandataire: Macquet, Christophe
(86) Numéro de dépôt international: PCT/FR2013/052555
(87) Numéro de publication internationale: WO 2014/064397

(56) Documents cités:
- WO-A2-2014/029948
- FR-A1- 2 918 893
- FR-A1- 2 956 818
- US-A1- 2010 196 293
- GAURAV KAITHWAS ET AL: "Linum usitatissimum (linseed/flaxseed) fixed oil: antimicrobial activity and efficacy in bovine mastitis", INFLAMMOPHARMACOLOGY, KLUWER ACADEMIC PUBLISHERS, DORDRECHT, NL, vol. 19, no. 1, 1 février 2011 (2011-02-01), pages 45-52, XP002688296, ISSN: 0925-4692, DOI: 10.1007/S10787-010-0047-3
- FIRAS A. AL-BAYATI: "Antibacterial Activity of Linum usitatissimum L. Seeds and Active Compound Detection", RAF. JOUR. SCI., vol. 18, no. 2, 2007, pages 27-36, XP002698035,
- NAND PRATIBHA ET AL: "Antimicrobial investigation of Linum usitatissimum for the treatment of acne.", NATURAL PRODUCT COMMUNICATIONS NOV 2011, vol. 6, no. 11, novembre 2011 (2011-11), pages 1701-1704, XP009169892, ISSN: 1934-578X
- XU Y ET AL: "Antifungal activity stability of flaxseed protein extract using response surface methodology", JOURNAL OF FOOD SCIENCE, WILEY-BLACKWELL PUBLISHING, INC, US, vol. 73, no. 1, 1 janvier 2008 (2008-01-01), pages M9-M14, XP009169879, ISSN: 0022-1147, DOI: 10.1111/J.1750-3841.2007.00576.X

## Description

La présente invention se situe dans le domaine cosmétique et pharmaceutique, et plus particulièrement dans le domaine de la dermatologie. La présente invention concerne un extrait de lin pour son utilisation pour la protection de la peau et des phanères des agressions microbiennes.

L'invention concerne encore un extrait de lin pour son utilisation pour augmenter le taux d'expression de peptides antimicrobiens.

Le terme « phanères » selon l'invention englobe l'ensemble des annexes kératiniques présentes à la surface du corps, en particulier les poils, les cils, les sourcils, les ongles et les cheveux.

La fonction principale de l'épiderme est d'assurer une barrière protectrice entre l'organisme et l'environnement extérieur. Au niveau de l'épiderme, deux types de barrières sont en jeu : une barrière physique constituée par la peau elle-même assurée grâce à la forte cohésion intercellulaire (desmosomes, jonctions serrées) et la résistance à l'abrasion des cellules kératinisées, et une barrière chimique formée par les sécrétions de la peau (sébum, sueur) qui permet de lutter contre les allergènes et les irritants et possède un rôle antibactérien grâce au pH acide de la couche cornée et à la présence d'enzymes hydrolytiques et de peptides antimicrobiens. Les cellules du système immunitaire forment une troisième barrière défensive capable d'éliminer les microorganismes qui seraient parvenus à passer à travers l'épiderme.

La peau, en contact direct avec l'environnement extérieur, est exposée à de nombreux microorganismes qui colonisent les couches superficielles de l'épiderme et les annexes (les follicules pileux, les glandes sébacées et les glandes sudoripares). La peau maintient un écosystème stable, favorable à certains microorganismes dont les exigences de croissance sont compatibles avec les conditions locales. Sur la peau humaine peuvent se trouver plus de mille espèces de bactéries et leur nombre total est estimé à 10¹² bactéries. La flore microbienne cutanée du sujet sain est une flore microbienne commensale qui comprend la flore cutanée résidente, et la flore cutanée transitoire.

La flore cutanée résidente est dominée par les espèces à Gram positif, notamment les bactéries du genre Staphylococcus (par exemple S. *epidermidis*), Corynébactérium (par exemple C. *minutissimum*) et Propionibacterium (par exemple, *P. acnes*)*.* D'autres bactéries de la flore résidente sont par exemple certains microcoques ou le genre Brevibacterium.

La flore cutanée de transit est plus polymorphe et peut comporter des germes potentiellement pathogènes, provenant du tube digestif ou du rhinopharynx ou encore de l'environnement. Elle comprend les entérocoques, le staphylocoque doré et des bactéries gram négatif (notamment des acinetobactéries, entérobactéries, des Pseudomonas) et des champignons (Par exemple *C. Albicans*)*.*

Les bactéries de la flore microbienne transitoire font généralement un séjour bref sur la peau où l'environnement leur est peu favorable. Cependant, elles peuvent se comporter en pathogènes opportunistes et causer des infections chez les individus ayant des systèmes de défense affaiblis, par exemple dans le cas de maladies systémiques (patients immunodéprimés, diabétiques) ou dans les cas d'une altération de la fonction barrière de la peau (psoriasis ou dermatite atopique).

Toute lésion de la peau conduit à un affaiblissement de ses moyens de défense naturels. La peau est alors sensible à des agressions de microorganismes pathogènes. De plus, les flores microbiennes cutanées résidentes ou transitoires peuvent alors se comporter en pathogènes opportunistes, coloniser la lésion puis, si les conditions sont favorables, provoquer un processus infectieux local et/ou systémique.

La flore microbienne commensale cutanée participe au maintien de l'écosystème et de la santé de la peau en participant à la fonction barrière chimique de la peau. En effet, les germes résidents de la peau jouent un rôle important dans la résistance à la colonisation de la peau par des microorganismes pathogènes car ils sont en compétition pour l'espace et la nourriture avec les bactéries pathogènes, libèrent des substances antibactériennes (inhibiteurs, création de conditions de pH défavorables ou modification de récepteurs) qui ralentissent la croissance des bactéries pathogènes et stimulent de manière continue le système immunitaire ce qui permet la régulation de la flore bactérienne de la peau et des phanères.

En outre, les kératinocytes et les sébocytes participent également à l'équilibre de la flore résidente cutanée et protègent la peau des microorganismes pathogènes en expriment, entre autres, des Peptides Antimicrobiens (AMPs). Les AMPs sont une famille de polypeptides de moins de 100 acides aminés qui ont un spectre d'activité contre les microorganismes pathogènes large, comprenant les bactéries, les champignons, les virus enveloppés et les protozoaires. Les mécanismes d'action des AMPs comprennent une activité antimicrobienne directe et l'initiation d'une réponse hôte qui entraîne la libération de cytokines, l'inflammation, l'angiogenèse et la re-épithélialisation. Le mécanisme de l'activité antimicrobienne directe des AMPs passe par leur fixation sur la membrane des microorganismes cibles sous forme de pores multimériques ce qui conduit à la lyse des microorganismes cible.

Chez l'homme, deux groupes d'AMPs sont majoritairement exprimés par la peau : les Defensines (DEFBs) et Cathélicidine épithéliales qui sont des médiateurs cationiques du système immunitaire inné non spécifique.

La Cathélicidine humaine hCAP18 est un propeptide inactif. Son fragment C-terminal LL37 a une activité antimicrobienne à large spectre d'action et module la réponse immunitaire. Dans l'épiderme, les Kallikréines, enzymes primordiales impliquées dans le processus de desquamation, sont responsables de la maturation de la cathélicidine en LL-37 post-transcriptionnellement.

Les défensines sont des petits peptides cationiques de moins de 50 acides aminés exprimés sous forme de prépropeptides précurseurs. La famille des défensines peut être divisée en deux groupes : les α-defensines (qui se trouvent dans les neutrophiles et les cellules de Paneth du petit intestin) et les β-défensines exprimées à la surface des cellules épithéliales. Six β-défensines nommées DEFB1 à 6 ont été identifies dans les tissus humains, parmi ceux-ci, les DEFB 1, 2 et 3 sont exprimés dans la peau. Les DEFB 1, 2 et 3 sont exprimés de manière constitutive par les kératinocytes des couches supérieures de la peau saine et participent à la barrière chimique de la peau en inhibant la croissance des microorganismes pathogènes et leur invasion de la surface de la peau.

Dans ce contexte, les propriétés des AMPs apparaissent comme particulièrement intéressantes pour renforcer la fonction protectrice de la peau contre les agressions microbiennes et pour favoriser l'équilibre de la microflore résidente de la peau.

Un certain nombre de substances introduites dans des produits cosmétiques ou pharmaceutiques à application topique ont vu le jour. Les documents US 2009/0005300 et US 2010/0215591 décrivent des AMPs naturels ou synthétiques et leur utilisation comme antimicrobiens dans des compositions pharmaceutiques ou cosmétiques. Cependant, il existe toujours un besoin, de développer de nouveaux ingrédients permettant de protéger la santé et l'intégrité des fonctions de la peau. En outre, il existe toujours un besoin, de développer de nouveaux ingrédients pour améliorer l'aspect esthétique de la peau, notamment pour les personnes ayant des peaux sensibles et irritables.

La présente invention a pour objectif de proposer un agent actif présentant l'avantage d'être d'origine naturelle et permettant de protéger la flore microbienne commensale de la peau. Les inventeurs ont mis en évidence une activité antimicrobienne, d'un extrait de lin, décrit dans la présente invention. Il a notamment été mis en évidence que cet extrait de lin, lorsqu'il est appliqué sur la peau, a une forte activité protectrice vis-à-vis de la flore bactérienne commensale de la peau et des agressions par les agents infectieux qui provoquent des réactions d'irritation de la peau.

Ce nouveau principe actif, capable d'augmenter le taux d'expression d'AMPs de la peau permet ainsi d'ouvrir de nouvelles perspectives cosmétiques et thérapeutiques.

Selon un premier aspect, un extrait de lin en tant qu'agent actif antimicrobien est décrit. L'invention et les avantages qui en découlent seront mieux compris à la lecture de la description.

L'invention s'adresse aux mammifères en général, et plus particulièrement, aux êtres humains.

Le terme « antimicrobien » ou « protection des agressions microbiennes » est relatif à la mort des microorganismes, à l'inhibition de leur croissance ou à la prévention de leur croissance. « L'inhibition de la croissance des microorganismes pathogènes » signifie la réduction de la croissance des microorganismes pathogènes, par exemple en termes de réduction du nombre de colonies et/ou de la taille des colonies de microorganismes pathogènes, tandis que le terme « prévention de la croissance des microorganismes » désigne l'arrêt de la croissance des microorganismes.

Par « microorganismes » ou « microbes » on entend tout organisme compris dans les domaines phylogénétiques des archées ou des bactéries, ainsi que les champignons unicellulaires et filamenteux (par exemple les levures), les algues unicellulaires ou filamenteuses, les parasites uni et pluri cellulaires et les virus.

Selon des caractéristiques particulières définies dans les revendications, l'invention concerne un extrait de lin pour son utilisation pour la protection de la peau et des phanères des agressions microbiennes.

L'utilisation de l'extrait de lin, ou d'une composition le comprenant, sur des peaux ayant la fonction barrière chimique altérée ou un affaiblissement du système immunitaire local, va permettre à la peau et aux phanères d'être protégés et de mieux résister aux agressions par des microorganismes pathogènes ou opportunistes.

Selon des caractéristiques particulières, un extrait de lin pour son utilisation pour augmenter le taux d'expression de peptides antimicrobiens est décrit.

De manière préférée, un extrait de lin pour augmenter le taux d'expression cellulaire de défensines et/ou de cathélicidine est décrit. Selon des caractéristiques particulières, un extrait de lin pour son utilisation pour stimuler les défenses immunitaires de la peau est décrit. Selon des caractéristiques particulières, l'invention concerne un extrait de lin pour son utilisation pour le traitement des infections par *Staphylococcus aureus* de la peau et des phanères.

L'invention se rapporte encore à un extrait de lin pour son utilisation pour le traitement des dermatites atopiques et/ou la rosacée de la peau.

La rosacée est une affection de la peau dont la cause est d'origine bactérienne, éventuellement associée à un terrain vulnérabilisant les systèmes de protection de la peau. La dermatite atopique est également une affection de la peau, dans laquelle l'expression de la cathélicidine est anormalement diminuée.

Avantageusement l'extrait de lin pour son utilisation selon l'invention permet de stimuler la production d'AMPs de type cathélicidine par les cellules de la peau, sans provoquer de réaction toxique ou allergique de la peau.

Selon un deuxième aspect, l'utilisation d'un extrait de lin pour renforcer la fonction barrière chimique de la peau et des phanères est décrite. On entend par «renforcer la fonction barrière chimique », que l'application sur des peaux saines et/ou des peaux sensibles d'un extrait de lin ou d'une composition cosmétique le comprenant permet d'augmenter le taux d'expression cellulaire des AMPs. L'extrait de lin a une action localisée au niveau de l'épiderme et il permet de calmer les irritations de la peau de type rougeurs ou inconforts des peaux sensibles.

Selon des caractéristiques particulières, l'utilisation d'un extrait de lin pour protéger la flore microbienne commensale et/ou à limiter le déséquilibre de la flore microbienne commensale de la peau et des phanères est décrite. Avantageusement, l'utilisation cosmétique de l'extrait de lin selon l'invention permet de maintenir une bonne fonction barrière chimique de la peau en stimulant localement au niveau de l'épiderme l'expression d'AMPs ce qui contribue au maintien de l'équilibre et des conditions favorables de l'écosystème de la microflore commensale de la peau.

L'extrait de lin utilisé selon l'invention est défini dans les revendications. L'extrait de lin provient de l'hydrolyse des protéines du lin.

De préférence encore, l'extrait de lin contient au moins 0,1 à 5 g/l de composés de nature peptidique en poids d'extrait sec, 0,1 à 2 g/l de sucres en poids d'extrait sec et comprend essentiellement des composés peptidiques de poids moléculaire inférieur à 5 kDa, de préférence de poids moléculaire inférieur à 2,5 kDa.

Le terme « peptide » désigne un enchaînement de deux ou plusieurs acides aminés liés entre eux par des liaisons peptidiques ou par des liaisons peptidiques modifiées ; le terme « polypeptide » désignant un peptide de taille plus importante ; le terme de « composés peptidiques » désignant les fragments de protéines, les peptides et les acides aminés libres présents dans le mélange.

Le terme « hydrolysat ou issu de l'hydrolyse » désigne toute substance ou mélange de substances, ou préparation isolée, obtenue après hydrolyse de matière végétale.

Pour réaliser l'extraction, on peut utiliser la plante entière, ou une partie spécifique de la plante (feuille, graine, etc.).

Plus particulièrement selon l'invention, on utilise une des nombreuses plantes de la famille des linacées, du genre *Linum* (lin). Le genre *Linum* compte près de 200 espèces poussant sur tout l'hémisphère nord. Ce sont des plantes herbacées à tiges fibreuses, à feuilles simples, aux fleurs à 5 pétales. Préférentiellement selon l'invention, on utilise l'espèce cultivée *Linum usitatissimum L.* L'extrait de lin (*Linum*) selon l'invention est préférentiellement un extrait peptidique provenant de l'hydrolyse des protéines extraites des graines de lin et préférentiellement la graine débarrassée de son enveloppe par une étape de décorticage.

Toute méthode d'extraction ou de purification connue de l'homme du métier peut être utilisée afin de préparer l'extrait de lin selon l'invention.

Préférentiellement, l'extrait de lin est obtenu par un procédé qui comprend :
- une étape d'extraction des protéines d'origine végétale,
- une étape d'hydrolyse contrôlée qui libère des composés peptidiques biologiquement actifs.

De très nombreuses protéines trouvées dans les plantes sont susceptibles de contenir des composés peptidiques biologiquement actifs au sein de leurs structures. L'hydrolyse ménagée permet de dégager ces composés peptidiques. Il est possible, mais non nécessaire pour réaliser l'invention, d'extraire soit les protéines concernées d'abord et de les hydrolyser ensuite, soit d'effectuer l'hydrolyse d'abord sur un extrait brut et de purifier les composés peptidiques ensuite.

Un mode particulier de mise en oeuvre du procédé d'obtention de l'extrait de lin selon l'invention est décrit ci-dessous.

Dans une première étape, la plante est broyée à l'aide d'un broyeur à plantes. La poudre ainsi obtenue peut ultérieurement être "délipidée" à l'aide d'un solvant organique classique (comme par exemple un alcool, l'hexane ou de l'acétone).

Dans une deuxième étape on réalise l'extraction des protéines de la plante suivant un procédé classique. Le broyat de plante est mis en suspension dans une solution alcaline contenant un produit adsorbant de type polyvinylpolypyrrolidone (PVPP) insoluble (0,01 - 20 %) ; en effet il a été observé que les opérations d'hydrolyses et de purifications ultérieures étaient facilitées par ce moyen. La concentration des substances de type phénoliques, interagissant avec les protéines, se trouve ainsi réduite.

La fraction soluble est recueillie après des étapes de centrifugation et de filtration, cette solution brute constituant alors une première forme de l'extrait comprenant les protéines, les glucides et éventuellement des lipides.

Selon un mode particulier de mise en oeuvre du procédé, les protéines peuvent être ensuite précipitées en faisant varier la force ionique en acidifiant le milieu, ce qui permet d'éliminer les composants solubles. Le précipité est ensuite lavé à l'aide d'un solvant organique tel que, par exemple, l'éthanol ou le butanol puis le solvant est évaporé par séchage sous vide. Le précipité riche en protéines est remis en solution dans l'eau ou un autre solvant et constitue alors une forme plus purifiée de l'hydrolysat.

L'étape d'extraction des protéines de la plante peut également être réalisée en milieu neutre ou acide toujours en présence de polyvinylpolypyrrolidone. Après une étape de filtration, une étape de précipitation peut être effectuée dans un mode particulier de mise en oeuvre, à l'aide d'un agent classique de précipitation tel que les sels (chlorure de sodium, sulfate d'ammonium) ou un solvant organique (alcool, acétone). Le précipité obtenu peut être séparé des agents de précipitation par dialyse après remise en solution dans de l'eau ou un autre solvant.

La fraction soluble, comprenant les protéines, des glucides et éventuellement des lipides, est recueillie après des étapes de centrifugation et de filtration. Cette solution brute est ensuite hydrolysée dans des conditions ménagées pour générer des composés peptidiques, des polypeptides et des peptides solubles. L'hydrolyse se définit comme étant une réaction chimique impliquant le clivage d'une molécule par de l'eau, cette réaction pouvant se faire en milieu neutre, acide ou basique. Selon l'invention, l'hydrolyse est réalisée par voie chimique et/ou de façon avantageuse par des enzymes protéolytiques.

Selon des caractéristiques particulières, l'hydrolyse est réalisée par une protéase ou un mélange de protéases et/ou une cellulase ou un mélange de cellulases.

Selon des caractéristiques préférées, on utilise un mélange d'enzymes comprenant :
- des endoprotéases d'origine végétale (par exemple papaïne, bromelaïne, ficine, etc.) et/ou issues de micro-organismes (Aspergillus, Rhizopus, Bacillus, Alcalase® etc.),
- et/ou une cellulase ou un mélange de cellulases (par exemple Celluclast® CL).

Avantageusement les cellulases permettent une meilleure hydrolyse de la paroi cellulaire du lin ce qui augmente l'accessibilité aux protéines et facilite la filtration. En effet, les cellulases permettent l'hydrolyse des parois cellulaires en oligosaccharides de petites tailles. L'action conjointe des cellulases et de protéases conduit alors à l'obtention de polypeptides de faibles poids moléculaires inférieurs à 5 kDa et plus particulièrement une fraction importante inférieure à 2,5 kDa. Une fois ces différentes hydrolyses effectuées, une étape de désactivation thermique est nécessaire pour inactiver ces enzymes.

Pour les mêmes raisons que précédemment, c'est-à-dire l'élimination des substances polyphénoliques, une quantité de polyvinylpolypyrrolidone est additionnée au milieu réactionnel lors de cette étape d'hydrolyse ménagée.

Après filtration, la solution obtenue constitue une première forme avantageuse d'extrait peptidique de lin selon l'invention. L'extrait peptidique de lin peut être encore purifié afin de sélectionner les poids moléculaires et la nature des peptides générés. Le fractionnement peut s'effectuer avantageusement par ultrafiltration et/ou par une méthode de type chromatographique.

L'utilisation d'extraits peptidiques, et en particulier d'extraits peptidiques de bas poids moléculaires, présente de nombreux avantages en cosmétique. Outre le fait de générer des composés peptidiques qui ne préexistaient pas dans le mélange protéique de départ, l'hydrolyse et la purification permettent d'obtenir un mélange de composés peptidiques plus stables, de composition plus facilement reproductible et ne provoquant pas de réactions allergiques en cosmétique.

Selon des caractéristiques particulières, l'extrait de lin selon l'invention comprend essentiellement des composés peptidiques de bas poids moléculaires. Préférentiellement, l'extrait peptidique de lin selon l'invention comprend essentiellement des composés peptidiques de poids moléculaire inférieur à 2,5 kDa.

L'une quelconque des formes plus ou moins purifiées de l'hydrolysat est alors solubilisée dans de l'eau ou dans tout mélange contenant de l'eau, puis stérilisée par ultrafiltration.

L'extrait de lin est analysé qualitativement et quantitativement pour ses caractéristiques physico-chimiques et sa teneur en composés peptidiques. On entend par composés peptidiques, les fragments de protéines, les peptides et les acides aminés libres présents dans le mélange. Les peptides, acides aminés et fragments de protéines sont dosés selon les techniques classiques, bien connues de l'homme du métier.

Ainsi, selon un mode de réalisation avantageux de l'invention, l'extrait de lin a un pH compris entre 4 et 5, et préférentiellement, entre 4 et 4,5. Avantageusement ce pH acide favorise la dissolution des protéines dans l'eau et stabilise les protéines. L'extrait de lin selon l'invention a un poids sec titrant entre 0,1 et 8 g/l, et de manière préférée entre 0,1 et 5 g/l, et comprend :
- entre 0,1 et 5 g/l de composés peptidiques en poids d'extrait sec,
- et entre 0,1 et 2 g/l de sucres en poids d'extrait sec.

L'extrait est alors dilué dans de l'eau ou dans tout mélange de solvant contenant de l'eau, puis stérilisée par filtration stérilisante (0,2 µm).

Selon des caractéristiques particulières, l'extrait est dilué dans un ou plusieurs solvants physiologiquement adaptés, tels que l'eau, le glycérol, l'éthanol, le propanediol, le butylène glycol, le dipropylène glycol, les diglycols éthoxylés ou propoxylés, les polyols cycliques ou tout mélange de ces solvants. On entend par «physiologiquement adapté» que le solvant choisi est approprié pour entrer en contact avec la peau sans provoquer de réactions de toxicité ou d'intolérance.

Préférentiellement, après dilution l'extrait de lin selon comprend :
- entre 1,5 et 3,5 g/l de composés peptidiques en poids d'extrait sec,
- et environ 0,3 g/l de sucres en poids d'extrait sec.

La teneur en sucres est encore plus préférentiellement inférieure à 0,3 g/l dans l'extrait de lin selon l'invention.

Après cette étape de dilution, l'extrait peut être encapsulé ou inclus dans un vecteur cosmétique ou pharmaceutique tels que les liposomes ou toutes autres microcapsules utilisées dans le domaine de la cosmétique ou adsorbé sur des polymères organiques poudreux, des supports minéraux comme les talcs et bentonites, et plus généralement solubilisé dans, ou fixé sur, tout vecteur physiologiquement adapté.

L'extrait de lin peut être utilisé dans une composition cosmétique ou pharmaceutique. La composition peut comprendre une quantité d'extrait de lin nécessaire afin d'obtenir le résultat recherché, à savoir : protéger la peau et les phanères des agressions microbiennes, protéger la flore bactérienne de la peau et des phanères, stimuler les défenses immunitaires de la peau et stimuler l'expression des AMPs.

Selon un mode de réalisation, la composition comprend un extrait de lin est destinée à être utilisée dans la protection de la flore microbienne commensale et/ou la limitation du déséquilibre de la flore microbienne commensale de la peu et des phanères. L'extrait de lin présent dans la composition selon l'invention contient, plus particulièrement, au moins 0, 1 à 5 g/l de composés peptidiques en poids d'extrait sec, 0,1 à 2 g/l de sucres en poids d'extrait sec et comprend essentiellement des composés peptidiques de poids moléculaire inférieur à 5 kDa. La composition selon l'invention est appliquée de préférence topiquement sur des peaux saines et/ou des peaux sensibles en tant que qu'agent actif dans un solvant physiologiquement adapté.

Selon un mode de réalisation avantageux de l'invention, l'extrait de lin est présent dans la composition à une concentration comprise entre 0,0001 % à 20 % environ, et préférentiellement à une concentration comprise entre 0,05 % et 5 % environ, encore plus préférentiellement à une concentration comprise entre 1 % et 3 % environ par rapport au poids total de la composition finale.

Les compositions peuvent être appliquées par toute voie appropriée, notamment orale, parentérale ou topique externe, et leurs formulations sont adaptées par l'homme du métier, en particulier pour des compositions cosmétiques ou pharmaceutiques.

Avantageusement, les compositions sont destinées à une administration, par voie topique cutanée, sur au moins une partie de la peau du visage ou du corps. La composition cosmétique selon l'invention peut être utilisée comme produit de soin et/ou comme produit de maquillage de la peau. Ces compositions doivent donc contenir un milieu physiologiquement adapté selon l'invention, c'est-à-dire un milieu qui convient à une utilisation en contact avec la peau ou les phanères humains, sans risque par exemple de toxicité, d'incompatibilité, d'instabilité, ou encore de réponse allergique.

De manière préférée, la composition est destinée à être appliquée de façon topique sur au moins une partie de la peau du visage ou du corps se présente sous forme d'une solution aqueuse, hydro-alcoolique ou huileuse, émulsion huile-dans-eau, ou eau-dans-huile ou émulsion multiple, solution, suspension, microémulsion, gel aqueux ou anhydre, sérum, ou encore de dispersion de vésicules, de colloïde. Ces compositions peuvent aussi se présenter sous forme de crèmes, de suspensions, ou encore de poudres, adaptées à une application sur la peau, les muqueuses, les lèvres et/ou les phanères. Ces compositions peuvent être plus ou moins fluides et avoir l'aspect d'une crème, d'une lotion, d'un lait, d'un sérum, d'une pommade, d'une crème, d'une pâte, d'un onguent, ou d'une mousse. Elles peuvent aussi se présenter sous forme solide, comme un stick, un patch, ou être appliquées sur la peau sous forme d'aérosol ou de spray. Elles peuvent être utilisées comme produit de soin et/ou comme produit de maquillage de la peau. Ces compositions peuvent également être adaptées à des applications sur le cuir chevelu et/ou les cheveux, et notamment un shampooing, un après-shampooing, une lotion de mise en plis, une lotion traitante, une crème ou un gel coiffant, une lotion pour les cheveux, un masque, etc. La composition cosmétique selon l'invention peut être utilisée notamment dans les traitements mettant en oeuvre une application qui est suivie ou non suivie d'un rinçage, ou encore sous forme de shampooing. Elle peut également se présenter sous forme de teinture ou de mascara à appliquer au pinceau ou au peigne, en particulier sur les cils, les sourcils ou les cheveux.

Ces compositions comprennent, en outre, tout additif communément utilisé dans le domaine d'application envisagé ainsi que les adjuvants nécessaires à leur formulation, tels que des co-solvants (éthanol, glycérol, alcool benzylique, humectant...), des épaississants, des diluants, des émulsionnants, des anti-oxydants, des colorants, des filtres solaires, des pigments, des charges, des conservateurs, des parfums, des absorbeurs d'odeur, des huiles essentielles, des oligo-éléments, des acides gras essentiels, des tensioactifs, des polymères filmogènes, des filtres chimiques ou minéraux, des agents hydratants ou des eaux thermales etc. On peut, par exemple, citer des polymères hydrosolubles de type polymère naturel, tels que les polysaccharides, ou polypeptides, des dérivés cellulosiques de type méthylcellulose ou hydroxypropylcellulose, ou encore des polymères synthétiques, polaxamères, carbomères, siloxanes, PVA ou PVP, et notamment les polymères vendus par la société Ashland. Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre à 0,01 à 20 % du poids total de la composition. Lorsque la composition de l'invention est une émulsion, la phase grasse peut représenter de 5 à 80 % en poids et de préférence de 5 à 30 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 10 % en poids, par rapport au poids total de la composition.

Il est bien entendu que le principe actif selon l'invention peut être utilisé seul ou bien en association avec au moins un autre principe actif, dans une composition cosmétique.

Avantageusement, les compositions utilisables selon l'invention peuvent comprendre en outre divers principes actifs destinés à favoriser l'action de l'agent actif selon l'invention, notamment, à la prévention et/ou au traitement des désordres liés à la fonction barrière de la peau ou à l'apaisement de la peau.

On peut citer, de manière non limitative, les classes d'ingrédients suivantes : d'autres principes actifs peptidiques, des extraits de végétaux, des agents cicatrisants, anti-âge, antirides, apaisants, anti-radicalaires, anti-UV, des agents stimulant la synthèse de macromolécules dermiques ou le métabolisme énergétique, des agents hydratants, antibactériens, antifongiques, anti-inflammatoires, anesthésiques, des agents modulants la différenciation, la pigmentation ou la dépigmentation cutanée, des agents stimulants la pousse des ongles ou des cheveux etc. Préférentiellement, on utilisera un agent présentant une activité apaisante, ou un agent stimulant la synthèse de macromolécules dermiques, ou encore un agent stimulant le métabolisme énergétique. Plus particulièrement, le principe actif est choisi parmi les principes actifs peptidiques bioactifs, les vitamines, les phytostérols, les flavonoïdes, la DHEA et/ou un de ses précurseurs ou un de ses dérivés chimiques ou biologiques, un inhibiteur de métalloprotéinase, ou un rétinoïde.

Selon un troisième aspect, un procédé de traitement cosmétique destiné à protéger la flore microbienne commensale et/ou à limiter le déséquilibre de la flore microbienne commensale, caractérisé en ce que l'on applique topiquement sur la peau ou les phanères à traiter une composition comprenant un extrait de lin est décrit.

Des modes de réalisation particuliers de ce procédé de traitement cosmétique résultent également de la description précédente. D'autres avantages et caractéristiques de l'invention apparaîtront mieux à la lecture des exemples donnés à titre illustratif et non limitatif.

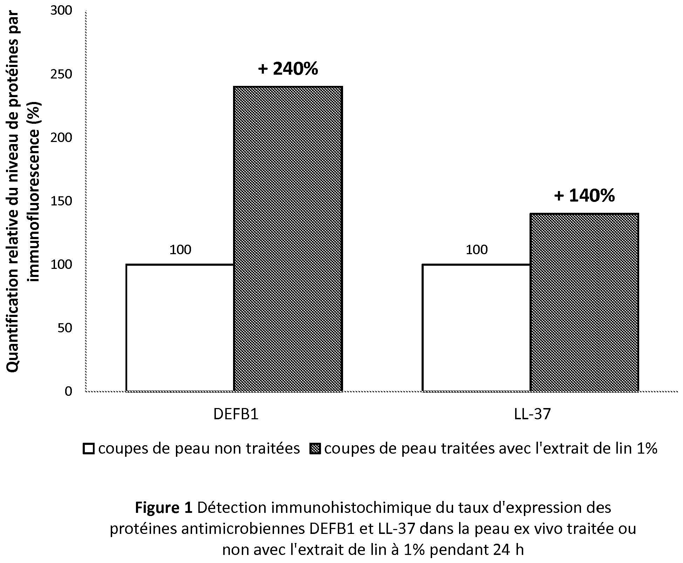

La Figure 1 montre sous la forme d'un histogramme la détection immunohistochimique du taux d'expression des protéines antimicrobiennes DEFB1 et LL-37 dans la peau ex vivo traitée ou non avec l'extrait de lin à 1% pendant 24 h

### Exemple 1 : Préparation de principe actif à partir de lin (Linum usitatissimum L.)

Dans une première étape, 1 kg de graines de lin (*Linum usitatissimum L.*) décortiquées sont broyées dans un broyeur à céréales. La farine obtenue (1 kg) est mise en présence de 10 litres d'hexane. Le mélange est ensuite agité 2 heures à température ambiance afin de procéder à une délipidation de la matière première. Après filtration et séchage sous vide, la poudre obtenue est mise en suspension dans 800 ml d'une solution aqueuse alcaline (dilution au 1/10) pH 10 contenant 1 % de polyvinylpolypyrrolidone (Polyclar V ISP). Ce mélange est maintenu sous agitation pendant 2 heures à température ambiante pour permettre la solubilisation des fractions solubles. Après cette phase d'extraction le milieu est clarifié par centrifugation puis filtré sur filtre à plaque. Ce filtrat qui contient les fractions solubles du lin est ensuite soumis à une précipitation des protéines en faisant varier la force ionique en milieu neutre ou acide, ce qui permet d'éliminer les composants glucidiques solubles, les lipides et les acides nucléiques le milieu est amené à pH 3,5. Le surnageant est éliminé et le précipité est ensuite lavé à l'aide d'éthanol puis le solvant est évaporé par séchage sous vide.

A ce stade, on obtient environ 50 grammes de poudre de couleur jaune clair d'extrait protéique brut contenant :
- Protéines : 78 %
- Glucides : 20 %
- Lipides < 2 %

Le précipité riche en protéines est remis en solution dans 500 grammes d'eau.

L'extrait protéique brut est alors soumis à une série d'hydrolyses enzymatiques ménagées et sélectives en présence de 0,5 % de PVPP (Polyclar V) et d'endopeptidases à cystéine (2 g/l bromélaine et 2 g/l alcalase). Après 2 heures de réaction à 50°C puis désactivation du cocktail enzymatique pendant 2 heures à 80°C, l'hydrolysat est filtré sur plaques de porosité décroissante puis sur cartouche stérilisante (0,2 µm).

On obtient alors un hydrolysat de couleur claire, titrant de 15 à 30 g/l d'extrait sec, qui est alors dilué de telle sorte que la concentration en composés peptidiques déterminée par la méthode de Lowry, soit comprise entre 0,1 et 5 g/l et préférentiellement entre 1,5 et 3,5 g/l. L'analyse physico-chimique de l'hydrolysat végétal, qui constitue le principe actif, montre que son pH est compris entre 4 et 5, et préférentiellement entre 4 et 4,5. L'extrait de lin selon l'invention a un extrait sec titrant entre 1 et 8 g/l, et de manière préférée entre 0,1 et 5 g/l, et comprend entre 0,1 et 5 g/l de composés peptidiques en poids d'extrait sec et entre 0,1 et 2 g/l de sucres en poids d'extrait sec, préférentiellement l'extrait de lin selon l'invention est dilué pour contenir entre 1,5 et 3,5 g/l de composés peptidiques en poids d'extrait sec et entre 0,1 et 0,3 g/l de sucres en poids d'extrait sec.

### Exemple 2 : Mise en évidence de l'effet activateur de l'extrait de lin selon l'exemple 1 sur le taux d'expression des ARN messagers de DEFB1 et de LL37 dans les kératinocytes

Le but de cette étude est de déterminer l'influence de l'extrait de lin selon l'exemple 1 sur l'expression du transcrit de DEFB1 et sur l'expression du transcrit de LL-37. Pour évaluer le taux d'expression de l'ARN messager de DEFB1 et le taux d'expression de l'ARN messager de LL-37, des quantifications par réaction en chaine par polymérase (PCR) en temps réel ont été réalisées.

### Protocole :

Des cellules NHK (kératinocytes humains primaires normaux) sont traitées avec l'extrait de lin à 1% selon l'exemple 1, deux fois par jour, durant 48 heures ou ne sont pas traitées (contrôle).

Afin d'évaluer le taux d'expression de l'ARN messager de DEFB1 et le d'expression de l'ARN messager de LL-37, il faut isoler les ARNs totaux des kératinocytes en culture, traités ou non par l'extrait de lin selon l'exemple 1. Les ARNs totaux sont ensuite transformés en ADN complémentaires par l'action d'une enzyme transcriptase inverse. Une quantification des ADN complémentaires est ensuite réalisée par PCR en temps réel à l'aide d'un thermocycleur StepOnePlus™ (Applied Biosystems). Cette quantification permet de déterminer le taux d'expression des ARNs messagers de DEFB1 et le taux d'expression des ARNs messagers de LL-37.

### Résultats :

On observe une augmentation de 31% du taux d'expression de l'ARN messager de DEFB1 et une augmentation de 23% du taux d'expression de l'ARN messager de LL-37, dans les cellules après 48 heures de traitement avec l'extrait de lin selon l'exemple 1, en comparaison avec les cellules non traitées.

### Conclusion :

L'extrait de lin selon l'exemple 1 augmente le taux d'expression de l'ARN messager codant pour DEFB1 et le taux d'expression de l'ARN messager codant pour LL-37, dans des kératinocytes humains normaux.

### Exemple 3 : Mise en évidence de l'effet activateur de l'extrait de lin selon l'exemple 1 sur l'expression de la protéine antimicrobienne DEFB1 et sur l'expression de la protéine antimicrobienne LL-37 dans les kératinocytes

Le but de cette étude est de déterminer l'influence de l'extrait de lin selon l'exemple 1 sur l'expression de la protéine antimicrobienne DEFB1 et l'expression de la protéine antimicrobienne LL-37. Pour cela, le taux d'expression de la protéine DEFB1 et le taux d'expression de la protéine LL-37 ont été évalués par immuno-cytochimie sur des kératinocytes traités pendant 24 heures avec l'extrait de lin selon l'exemple 1 à 1%, ou sans traitement (contrôle) et inclus en paraffine.

### Protocole :

Des cellules NHK (kératinocytes humains primaires normaux) sont cultivées. Ces cellules sont ensuite fixées au formol à 10% puis inclues dans de la paraffine. Le bloc cellulaire est ensuite coupé en sections de 4 µm d'épaisseur avec un microtome et sont ensuite transférées sur une lame.

Les coupes sont déparaffinées dans du xylène 100 %, puis réhydratées dans des bains d'alcool successifs: 2 bains d'éthanol (EtOH) 100% pendant 2 minutes, 1 bain d'EtOH 95% pendant 2 minutes, 1 bain d'EtOH 90% pendant 2 minutes et 1 bain d'H₂O pendant 5 minutes. Les lames sont plongées dans un tampon d'acide citrique à 0,01 M pH 6 et chauffées jusqu'à légère ébullition afin de faciliter l'accès de l'anticorps à la protéine d'intérêt. Les coupes sont incubées avec 5 % de BSA pendant 30 minutes, puis avec l'anticorps primaire : anticorps polyclonal de lapin anti -"DEFB1" ab14425 (Abcam, Cambridge, UK), dilué au 1/500 dans du PBS ou anticorps monoclonal de souris anti -"LL-37" sc-166770 (Tebu Santa Cruz, CA, USA) dilué au 1/75 dans du PBS, pendant 1 heure et demie sous agitation et à température ambiante. Après plusieurs lavages au PBS, les coupes sont incubées avec l'anticorps secondaire fluorescent (Anticorps Alexa Fluor 488 anti-lapin A21206 (Invitrogen, Fisher)) dilué au 1/1000 dans du PBS pendant 1 heure à température ambiante. Les noyaux cellulaires sont marqués avec 0,3 µM de 4',6'-diamidino-2-phénylindole (DAPI) (Molecular Probes). Les coupes sont rincées pendant 5 minutes dans du PBS. L'expression de la protéine antimicrobienne DEFB1 et de la protéine antimicrobienne LL-37 est détectée au moyen d'un microscope à fluorescence (objectif 40x).

### Résultats :

Les résultats montrent qu'il y a une augmentation de 118% de l'expression de la protéine antimicrobienne DEFB1 et une augmentation de 136% de l'expression de la protéine antimicrobienne LL-37 dans des kératinocytes traités avec l'extrait de lin selon l'exemple 1 à 1% après 24 heures, en comparaison à des kératinocytes non traités.

### Conclusion :

L'extrait de lin selon l'exemple 1 stimule l'expression de la protéine antimicrobienne DEFB1 et l'expression de la protéine antimicrobienne LL-37 dans les kératinocytes.

### Exemple 4 : Mise en évidence de l'effet activateur de l'extrait de lin selon l'exemple 1 sur l'expression de la protéine antimicrobienne DEFB1 et sur l'expression de la protéine antimicrobienne LL-37 dans la peau ex vivo

Le but de cette étude est de déterminer l'influence de l'extrait de lin selon l'exemple 1 sur l'expression de DEFB1 et sur l'expression de LL-37. Pour évaluer le niveau d'expression de DEFB1 et de LL-37, un immunomarquage de DEFB1 et de LL-37 sur coupes de peau *ex vivo* a été réalisée.

### Protocole :

Des biopsies de peau humaine sont maintenues en culture *ex vivo,* puis traitées, deux fois par jour pendant 24 heures, par application topique de 20 µl d'une solution à 1 % de l'extrait de lin selon l'exemple 1 ou non traitées.

Les biopsies de peau sont ensuite fixées puis inclues en paraffine après passage dans un automate Shandon Hypercenter XP (Shandon, UK). Les biospies de peau incluses en paraffine sont ensuite coupées en sections de 4 µm d'épaisseur avec un microtome qui sont elles-même transférées sur une lame. Les coupes sont déparaffinées dans du xylène 100 %, puis réhydratées dans des bains d'alcool successifs: 2 bains d'éthanol (EtOH) 100% pendant 2 minutes, 1 bain d'EtOH 95% pendant 2 minutes, 1 bain d'EtOH 90% pendant 2 minutes et 1 bain d'H₂O pendant 5 minutes. Les lames sont plongées dans un tampon d'acide citrique à 0,01 M pH 6 et chauffées jusqu'à légère ébullition afin de faciliter l'accès de l'anticorps à la protéine d'intérêt. Les coupes sont incubées avec 5 % de BSA pendant 30 minutes, puis avec l'anticorps primaire : anticorps polyclonal de lapin anti -"DEFB1" ab14425 (Abcam, Cambridge, UK) dilué au 1/500 dans du PBS, ou anticorps monoclonal de souris anti -"LL-37" sc-166770 (Tebu-Santa Cruz, CA, USA) dilué au 1/100 dans du PBS, pendant 1 heure et demie sous agitation et à température ambiante. Après plusieurs lavages au PBS, les coupes sont incubées avec l'anticorps secondaire fluorescent (Anticorps Alexa Fluor 488 anti-lapin A21206 (Invitrogen, Fisher)) dilué au 1/1000 dans du PBS pendant 1 heure à température ambiante. Les noyaux cellulaires sont marqués avec 0,3 µM de 4',6'-diamidino-2-phénylindole (DAPI) (Molecular Probes). Les lames sont rincées pendant 5 minutes dans du PBS. L'expression de la protéine DEFB1 et de la protéine LL-37 est détectée au moyen d'un microscope à fluorescence (objectif 40x).

### Résultats :

L'ensemble des résultats est montré dans la figure 1.

L'évaluation de la fluorescence obtenue sur les coupes par immuno-histochimie montre que les biopsies de peau traitées avec l'extrait de lin selon l'exemple 1 présentent un taux supérieur hautement significatif statistiquement (+240%) de l'expression de DEFB1 et (+140%) de l'expression de LL-37 en comparaison avec les biopsies de peau non traitées.

### Conclusion :

Un effet positif sur l'expression de la protéine antimicrobienne DEFB1 et sur l'expression de la protéine antimicrobienne LL-37 est obtenu suite au traitement avec de l'extrait de lin selon l'exemple 1 sur des biopsies de peau humaine.

### Exemple 5 : Mise en évidence de l'effet de l'extrait de lin selon l'exemple 1 sur le nombre et l'activité sécrétrice des corps lamellaires dans la peau ex vivo

Le but de cette étude est de déterminer l'influence de l'extrait de lin selon l'exemple 1 sur le nombre des corps lamellaires, lieu de stockage des protéines antimicrobiennes, présents à l'interface des couches granuleuses et cornées. Pour observer les corps lamellaires, des images de microscopie électronique provenant de coupe de peau *ex vivo* ont été réalisées.

### Protocole :

Des biopsies de peau humaine sont maintenues en culture *ex vivo,* puis traitées, une fois par jour pendant 24 heures, par application topique de 20 µl d'une solution à 1 % de l'extrait de lin selon l'exemple 1 ou non traitées.

Les biopsies de peau sont ensuite fixées à l'aide du tampon de fixation de Karnovsky (Electron Microscopy Sciences, Hatfield, UK) pendant 1 heure à température ambiante, puis toute la nuit à 4°C. Après avoir été rincées par un tampon de Cacodylate de Sodium à 0,1 M (Sigma, Steinheim, Germany), les biopsies sont post-fixées dans du tétroxide d'Osmium OsO4 à 1% pendant 1 heure, puis rincées et déshydratées dans des bains d'alcool successifs. Les échantillons sont infiltrés et inclus dans un mélange Epon-Epoxy à faible viscosité.

Les coupes sont réalisées à l'aide d'un microtome pourvu de couteau en diamant. Les coupes sont ensuite colorées avec de l'acétate d'uranyl et du citrate de plomb. L'observation s'effectue grâce à un microscope électronique à transmission à 60 keV.

### Résultats :

L'observation des images de microscopie électronique montre que les biopsies de peau traitées avec l'extrait de lin selon l'exemple 1 présentent un nombre plus important de corps lamellaires à l'interface des couches granuleuses et cornées en comparaison avec les biopsies de peau non traitées.

### Conclusion :

Un effet positif sur le nombre de corps lamellaires, lieu de stockage des peptides antimicrobiens, est observé suite au traitement avec de l'extrait de lin selon l'exemple 1 sur des biopsies de peau humaine.

### Exemple 6 : Mise en évidence de l'effet antimicrobien induit par l'extrait de lin selon l'exemple 1 dans les kératinocytes sur la croissance bactérienne de Staphylococcus aureus

Le but de cette étude est de déterminer l'effet d'une synthèse accrue de protéines antimicrobiennes produite par les kératinocytes suite à une stimulation par l'extrait de lin selon l'exemple 1 sur la croissance bactérienne de *Staphylococcus aureus.* Pour cela, un test de diffusion radiale a été réalisé.

### Protocole :

Des cellules NHK (kératinocytes humains primaires normaux) sont traitées avec l'extrait de lin selon l'exemple 1, deux fois par jour, pendant 24 heures ou non traitées (contrôle). Afin de réaliser un contrôle négatif, du milieu de culture de NHK n'ayant eu aucun contact avec les cellules est utilisé. Le surnageant des cellules est ensuite récupéré et 150 µl sont utilisés afin d'imbiber un disque de 6 mm de diamètre. Les disques sont ensuite placés sur une culture de *S*. *aureus* ensemencé dans la masse (4 disques par conditions et par boîte de Pétri). Après 48 heures, une zone de diffusion radiale est observée. Des photographies des zones d'inhibition sont prises avec la caméra QImaging Micropublisher 3.3 RTV et leurs diamètres sont calculés par le logiciel Q-capture Pro7™.

### Résultats :

Les résultats montrent qu'il y a une augmentation de 19% du diamètre de la zone d'inhibition de la croissance bactérienne suite à l'application de surnageant issu de culture de kératinocytes traités avec l'extrait de lin selon l'exemple 1 à 1% après 24 heures, en comparaison à des kératinocytes non traités.

### Conclusion :

L'extrait de lin selon l'exemple 1 permet une inhibition de la croissance bactérienne.

### Exemple 7 : Mise en évidence de l'effet antimicrobien induit par l'extrait de lin selon l'exemple 1 dans les kératinocytes sur la croissance bactérienne de Staphylococcus aureus

Le but de cette étude est de déterminer l'effet d'une synthèse accrue de protéines antimicrobiennes produite par les kératinocytes suite à une stimulation par l'extrait de lin selon l'exemple 1 sur la croissance bactérienne de *Staphylococcus aureus.* Pour cela, un test d'inhibition de la croissance bactérienne a été réalisé.

### Protocole :

Des cellules NHK (kératinocytes humains primaires normaux) sont traitées avec l'extrait de lin selon l'exemple 1, deux fois par jour, pendant 48 heures ou non traitées (contrôle). Afin de réaliser un contrôle négatif, du milieu de culture de NHK n'ayant eu aucun contact avec les cellules est utilisé. Le surnageant des cellules est ensuite récupéré et 200 µl sont utilisés et contaminés par une solution de *S*. *aureus.* Ce mélange surnageant/*S*. *aureus* est incubé pendant 3 heures à 37°C puis étalé sur des boites de gélose TSA. Après 48 heures, des colonies de *S*. *aureus* se sont développées et sont visibles à l'oeil nu. Des photographies des boites sont prises avec la caméra QImaging Micropublisher 3.3 RTV.

### Résultats :

Les résultats montrent qu'il y a une diminution de 79% de nombre de colonies S. *aureus* développées dans la condition où le surnageant a été issu de culture de kératinocytes traités avec l'extrait de lin selon l'exemple 1 à 1% après 24 heures, en comparaison à des kératinocytes non traités.

### Conclusion :

L'extrait de lin selon l'exemple 1 permet une diminution du nombre de colonies bactériennes.

### Exemples: Préparation de compositions

### Crème protectrice apaisante de jour :

| ***Noms commerciaux*** | ***Noms INCI*** | **% *massique*** |
|---|---|---|
| ***Phase A*** | | |
| Emulium Delta | Cetyl alcohol (and) Glyceryl Stearate (and) PEG-75 Stearate (and) Ceteth-20 (and) Steareth-20 | 4,00 |
| Lanette O | Cetearyl Alcohol | 1,50 |
| D C 200 Fluid/100cs | Dimethicone | 1,00 |
| DUB 810C | Coco Caprylate/Caprate | 1,00 |
| DPPG | Propylene Glycol Dipelargonate | 3,00 |
| DUB DPHCC | Dipentaerythrityl Hexacaprylate/Hexacaprate | 1,50 |
| Cegesoft PS6 | Vegetable Oil | 1,00 |
| Vitamine E | Tocopherol | 0,30 |

| ***Phase B*** | | |
|---|---|---|
| Eau déminéralisée | Aqua | qsp 100 |
| Glycerine | Glycerin | 2,00 |
| Carbopol EDT 2020 | Acrylates/C10-30Alkyl Acrylate Crosspolymer | 0,15 |
| Keltrol BT | Xanthan Gum | 0,30 |
| Allantoïne | Allantoin | 0.5 |

| ***Phase C*** | | |
|---|---|---|
| Sodium Hydroxide (sol.à 10 %) | Sodium Hydroxide | 0,30 |

| ***Phase D*** | | |
|---|---|---|
| Eau déminéralisée | Aqua | 5,00 |
| Stay-C 50 | Sodium Ascorbyl Phosphate | 0,50 |

| ***Phase E*** | | |
|---|---|---|
| Butylene Glycol | Butylene Glycol | 2,00 |
| ROKONSAL MEP | Phenoxyethanol (and) Methylparaben (and) Ethylparaben (and) Propylparaben | 1 |
| Dekaben CP | Chlorphenesin | 0,20 |
| Phase F | | |
| GP4G | Water (and) Artemia Extract | 1,00 |
| Extrait de lin selon l'exemple 1 | | 1,5 |

Préparer la phase A et chauffer à 75°C. Préparer la phase B en dispersant le carbopol, puis la gomme xanthane sous agitation. Laisser reposer jusqu'à parfaite homogénéité. Chauffer B à 75°C.

A 75°C, émulsionner A dans B sous agitation rotor-stator. Neutraliser avec la phase C sous agitation rapide. Après refroidissement à 40°C, additionner la phase D limpide, puis la phase E (préchaufée à 40°C et homogénéisée jusqu'à parfaite limpidité). Le refroidissement est poursuivi sous agitation légère jusqu'à 25°C et la phase F rajoutée.

### 2 -Lait de corps :

| Noms commerciaux | Noms INCI | % massique |
|---|---|---|
| PHASE A | | |
| Montanov L | C14-22 Alcohols (and) C12-20 Alkyl Glucoside | 3,00 |
| Waglinol 2559 | Cetearyl Isononanoate | 4,00 |
| Tegosoft TN | C12-15 Alkyl Benzoate | 3,00 |
| Huile de Noyaux d'Abricot | Prunus Armeniaca (Apricot) Kernel Oil | 2,00 |
| Huile d'Avocat | Persea Gratissima (Avocado) Oil | 1,00 |
| Abil 350 | Dimethicone | 1,00 |

| PHASE B | | |
|---|---|---|
| Eau déminéralisée | Aqua (Water) | Qsp 100 |
| Allantoïne | Allantoin | 0.5 |

| PHASE C | | |
|---|---|---|
| Simulgel EG | Sodium Acrylate/Acryloyldimethyl Taurate Copolymer (and) Isohexadecane (and) Polysorbate 80 Copolymer (and) Polysorbate 80 | 0,4 |

| PHASE D | | |
|---|---|---|
| ROKONSAL MEP | Phenoxyethanol (and) Methylparaben (and) Ethylparaben (and) Propylparaben | 1 |

| Noms commerciaux | Noms INCI | % massique |
|---|---|---|
| Germall 115 | Imidazolidinyl Urea | 0,20 |

| PHASE E | | |
|---|---|---|
| Extrait de lin selon l'exemple 1 | | 1 |

Les constituants de la phase A et de la phase B sont chauffés séparément entre 70°C et 75°C. La phase A est émulsionnée dans la phase B sous agitation. La phase C est ajoutée, à 45°C, en augmentant l'agitation. Les phases D et E sont ensuite additionnées lorsque la température se situe en dessous de 40°C. Le refroidissement est poursuivi jusqu'à 25°C sous vive agitation.

## Revendications

1. Extrait de lin provenant de l'hydrolyse des protéines du lin destiné à être utilisé en tant qu'agent actif antimicrobien dans le traitement des irritations de la peau et des phanères causées par des agressions microbiennes.

2. Extrait de lin provenant de l'hydrolyse des protéines du lin destiné à être utilisé dans le traitement de la dermatite atopique.

3. Extrait de lin provenant de l'hydrolyse des protéines du lin destiné à être utilisé dans la protection de la peau et des phanères contre des agressions microbiennes.

4. Extrait de lin provenant de l'hydrolyse des protéines du lin destiné à être utilisé dans le traitement des infections par *Staphylococcus aureus* de la peau et des phanères.

5. Extrait de lin destiné à être utilisé selon l'une des revendications précédentes, **caractérisé en ce que** l'extrait de lin contient au moins 0,1 à 5 g/l de composés peptidiques en poids d'extrait sec, 0,1 à 2 g/l de sucres en poids d'extrait sec et comprend essentiellement des composés peptidiques de poids moléculaire inférieur à 5 kDa.

6. Extrait de lin provenant de l'hydrolyse des protéines du lin destiné à être utilisé dans la protection de la flore microbienne commensale et/ou la limitation du déséquilibre de la flore microbienne commensale de la peau et des phanères.

7. Extrait de lin destiné à être utilisé selon l'une des revendications 6, **caractérisé en ce que** l'extrait de lin contient au moins 0, 1 à 5 g/l de composés peptidiques en poids d'extrait sec, 0,1 à 2 g/l de sucres en poids d'extrait sec et comprend essentiellement des composés peptidiques de poids moléculaire inférieur à 5 kDa.

8. Composition comprenant un extrait de lin destiné à être utilisé selon l'une des revendications 6 à 7, **caractérisé en ce que** ladite composition est appliquée topiquement sur des peaux saines et/ou des peaux sensibles en tant qu'agent actif dans un solvant physiologiquement adapté.

## Patentansprüche

1. Leinsamenextrakt, der aus der Hydrolyse von Proteinen des Leinsamens hervorgeht und dazu vorgesehen ist, als antimikrobieller Wirkstoff bei der Behandlung von Reizungen der Haut und Hautanhangsgebilde, die von mikrobiellen Belastungen verursacht werden, verwendet zu werden.

2. Leinsamenextrakt, der aus der Hydrolyse von Proteinen des Leinsamens hervorgeht und dazu vorgesehen ist, bei der Behandlung von atopischer Dermatitis verwendet zu werden.

3. Leinsamenextrakt, der aus der Hydrolyse von Proteinen des Leinsamens hervorgeht und dazu vorgesehen ist, beim Schutz der Haut und Hautanhangsgebilde vor mikrobiellen Belastungen verwendet zu werden.

4. Leinsamenextrakt, der aus der Hydrolyse von Proteinen des Leinsamens hervorgeht und dazu vorgesehen ist, bei der Behandlung von Infektionen der Haut und Hautanhangsgebilde mit *Staphylococcus aureus* verwendet zu werden.

5. Leinsamenextrakt, der dazu vorgesehen ist, nach einem der vorhergehenden Ansprüche verwendet zu werden, **dadurch gekennzeichnet, dass** der Leinsamenextrakt mindestens 0,1 bis 5 g/l peptidische Verbindungen, bezogen auf das Gewicht des trockenen Extrakts, 0,1 bis 2 g/l Zucker, bezogen auf das Gewicht des trockenen Extrakts, enthält und im Wesentlichen peptidische Verbindungen mit einem Molekulargewicht von weniger als 5 kDa umfasst.

6. Leinsamenextrakt, der aus der Hydrolyse von Proteinen des Leinsamens hervorgeht und dazu vorgesehen ist, beim Schutz der mikrobiellen Begleitflora und/oder beim Beschränken des Ungleichgewichts der mikrobiellen Begleitflora der Haut und Hautanhangsgebilde verwendet zu werden.

7. Leinsamenextrakt, der dazu vorgesehen ist, nach einem der Ansprüche 6 verwendet zu werden, **dadurch gekennzeichnet, dass** der Leinsamenextrakt mindestens 0,1 bis 5 g/l peptidische Verbindungen, bezogen auf das Gewicht des trockenen Extrakts, 0,1 bis 2 g/l Zucker, bezogen auf das Gewicht des trockenen Extrakts, enthält und im Wesentlichen peptidische Verbindungen mit einem Molekulargewicht von weniger als 5 kDa umfasst.

8. Zusammensetzung, die einen Leinsamenextrakt umfasst, der dazu vorgesehen ist, nach einem der Ansprüche 6 bis 7 verwendet zu werden, **dadurch gekennzeichnet, dass** die besagte Zusammensetzung in einem physiologisch geeigneten Lösungsmittel als Wirkstoff topisch auf gesunde Haut und/oder empfindliche Haut aufgetragen wird.

## Claims

1. Flax extract originating from the hydrolysis of flax proteins intended for use as an antimicrobial agent in the treatment of irritations of the skin and of the appendages caused by microbial attack.

2. Flax extract originating from the hydrolysis of flax proteins intended to be used in the treatment of atopic dermatitis.

3. Flax extract originating from the hydrolysis of flax proteins intended to be used in the protection of the skin and of the appendages against microbial attacks.

4. Flax extract originating from the hydrolysis of flax proteins intended to be used in the treatment of infections by Staphylococcus aureus of the skin and of the appendages.

5. Flax extract intended to be used according to one of the preceding claims, **characterised in that** the flax extract contains at least 0.1 to 5 g/l of peptide compounds by weight of dry substance, 0.1 to 2 g/l of sugars by weight of dry substance and substantially comprises peptide compounds with a molecular weight of less than 5 kDa.

6. Flax extract originating from the hydrolysis of flax proteins intended to be used in the protection of the commensal microbial flora and/or the limitation of the imbalance of the commensal microbial flora of the skin and of the appendages.

7. Flax extract intended to be used according to one of claims 6, **characterised in that** the flax extract contains at least 0.1 to 5 g/l of peptide compounds by weight of dry substance, 0.1 to 2 g/l of sugars by weight of dry substance and comprises substantially peptide compounds with a molecular weight of less than 5 kDa.

8. Composition comprising a flax extract intended to be used according to one of claims 6 to 7, **characterised in that** said composition is applied topically on health skin and/or on sensitive skin as an active agent in a physiologically adapted solvent.
